# EUROPÄISCHE PATENTSCHRIFT

(11) **EP 0 906 903 B1**
(45) Veröffentlichungstag und Bekanntmachung des Hinweises auf die Patenterteilung: **12.12.2001**
(21) Anmeldenummer: 98117341.2
(22) Anmeldetag: 12.09.1998
(51) Int. Cl.: C07C 217/90, C07D 239/52

(54) **Bis-o-amino(thio)phenole und deren Herstellung**
Bis-o-amino(thio)phenols and their preparartion
Bis-o-amino(thio)phénoles et leur préparation

(30) Priorität: 24.09.1997 DE 19742195
(43) Veröffentlichungstag der Anmeldung: 07.04.1999
(73) Patentinhaber: Infineon Technologies AG, 81669 München (DE)
(72) Erfinder: Sezi, Recai, Dr., 91341 Röttenbach (DE); Keitmann, Michael, 91074 Herzogenaurach (DE)
(74) Vertreter: Müller . Hoffmann & Partner Patentanwälte

(56) Entgegenhaltungen:
- EP-A- 0 023 662
- EP-A- 0 300 326
- DE-A- 19 537 893
- CHEMICAL ABSTRACTS, vol. 106, no. 10, 9. März 1987 Columbus, Ohio, US; abstract no. 67818, IGNATENKO, N. M. ET AL: "Bis(3-amino-4-hydroxyphenoxy)perfluoroary lenes as monomers for producing polybenzoxazoles with higher thermal and hydrolytic stability" XP002088350 & SU 1 205 518 A (INSTITUTE OF PHYSICAL-ORGANIC CHEMISTRY AND COAL CHEMISTRY, KIEV, USSR)

## Beschreibung

Die Erfindung betrifft neue Bis-o-aminophenole und Bis-o-aminothiophenole, die zusammen kurz auch als Bis-o-amino-(thio)phenole bezeichnet werden, sowie ein Verfahren zu deren Herstellung.

Bis-o-aminophenole werden insbesondere zur Herstellung von hochtemperaturstabilen Polymeren, wie Polybenzoxazolen (PBO) bzw. deren Vorstufen, sowie zur Herstellung von Hydroxypoly-imiden benötigt (siehe dazu beispielsweise EP 0 264 678 B1 und EP 0 300 326 B1). PBO-Vorstufen können in der Weise hergestellt werden, daß ein Dicarbonsäurechlorid mit einem Bis-o-aminophenol umgesetzt wird. Während aber wegen der Vielfalt industrieller Einsatzmöglichkeiten zahlreiche Dicarbonsäuren bzw. deren Chloride verfügbar sind, gibt es vergleichsweise wenige Bis-o-aminophenole. Außerdem beeinflußt die Art des eingesetzten Aminophenols in starkem Maße das Eigenschaftsspektrum des damit hergestellten Polymers. So werden nicht nur das thermische, elektrische und mechanische Verhalten, sondern auch die Löslichkeit und die Hydrolysestabilität sowie zahlreiche weitere Eigenschaften des Polymers durch das bei der Herstellung verwendete Aminophenol stark beeinflußt.

PBO-Vorstufen können in Form einer photosensitiven Zusammensetzung kostengünstig auf direktem Weg, d.h. ohne einen Hilfsresist, strukturiert werden. Im Vergleich mit anderen direkt photostrukturierbaren Dielektrika, wie Polyimid (PI) und Benzocyclobuten (BCB), bieten PBO-Vorstufen den Vorteil der positiven Strukturierbarkeit und der wäßrig-alkalischen Entwicklung (siehe EP 0 023 662 B1 und EP 0 264 678 B1). Hierzu müssen die verwendeten PBO-Vorstufen bei der Belichtungswellenlänge weitgehend transparent und im - vorzugsweise metallionenfreien - Entwickler ausreichend löslich sein. Wie die Polyimide haben auch Polybenzoxazole den großen Vorteil, daß sie - im Vergleich zum cyclisierten Endprodukt - als gut lösliche Vorstufe auf ein Substrat aufgebracht und anschließend cyclisiert werden können, wobei die Löslichkeit und damit die Sensibilität gegenüber Lösemitteln und anderen Prozeßchemikalien stark abnimmt.

Für den Einsatz von Polybenzoxazolen in der Mikroelektronik, insbesondere als Dielektrikum zwischen zwei Metallebenen, beispielweise bei Multi-Chip-Modulen sowie Speicher- und Logikchips, oder als Pufferschicht ("Buffercoat") zwischen dem Chip und seinem Gehäuse, ist neben guten elektrischen, mechanischen und thermischen Eigenschaften auch eine geringe Feuchteaufnahme erforderlich; der Feuchteanteil in der Polymerschicht beeinträchtigt nämlich einerseits die elektrischen Eigenschaften des Polymers und kann andererseits bei hohen Temperaturen zu Blasenbildungen und Abplatzungen führen. Ein gutes Planarisierungsvermögen der Polybenzoxazole ist ebenfalls von Vorteil. Durch die Verwendung eines gut planarisierenden Dielektrikums können nämlich bei der Herstellung von Bauteilen kostenintensive Schleifprozeduren (Chemical Mechanical Polishing; CMP) vermieden werden.

Aminophenole, die zur Herstellung von gut löslichen PBO-Vorstufen geeignet sind, sind beispielsweise aus der US-PS 4 525 539 und der EP 0 317 942 A2 bekannt. Hierbei gibt es jedoch keine Hinweise auf die Feuchteaufnahme oder das Planarisierungsverhalten der hergestellten Polymere nach der Cyclisierung auf dem Substrat (siehe EP 0 264 678 B1 und EP 0 317 942 A2). Bei der Herstellung der Aminophenole wird eine phenolische Ausgangsverbindung nitriert. Wenn dabei die Nitrierung nicht vollständig, d.h. zu 100 %, und vollkommen isomerenfrei erfolgt, d.h. nur in der o-Stellung zur Hydroxygruppe darf eine Nitrierung erfolgen, dann entstehen nach der Reduktion der Nitrogruppe teilweise Aminophenole, die in der PBO-Vorstufe keine vollständige Cyclisierung mehr zulassen und die Eigenschaften des Polybenzoxazols erheblich beeinträchtigen. Dies ist ein großer Nachteil der bekannten Herstellungsverfahren.

Aus der SU 1 205 518 A sind aromatische Aminophenole bekannt. Bei der Herstellung dieser Aminophenole wird cancerogenes Hydrazinhydrat verwendet, was einen erheblichen Nachteil darstellt. Außerdem gibt es auch hier keine Hinweise auf die Feuchteaufnahme und das Planarisierungsverhalten der hergestellten Polymeren nach der Cyclisierung auf dem Substrat.

Ein Verfahren zur Herstellung von Bisaminophenolen ist auch aus "Polymer Preprints" 34 (1), 1993, Seiten 425 und 426 bekannt. Dieses Verfahren hat den Nachteil, daß es hohe Temperaturen erfordert, d.h. deutlich höhere Temperaturen als 100°C (Lösungen in Dimethylacetamid und Toluol werden zum Rückfluß erhitzt). Hohe Reaktionstemperaturen fördern jedoch Nebenreaktionen, welche die Ausbeute verringern (sie liegt bei maximal 73 %) und die Reinigung des erwünschten Produktes erschweren. Außerdem sind die hergestellten Bisaminophenole nicht oxidationsstabil. Hierbei gibt es ebenfalls keine Hinweise auf die Feuchteaufnahme oder das Planarisierungsverhalten der hergestellten Polymeren nach der Cyclisierung auf dem Substrat.

Aufgabe der Erfindung ist es, Bis-o-aminophenole und Bis-o-aminothiophenole bereitzustellen, die zur Herstellung von Polymeren geeignet sind, welche die stark gestiegenen Anforderungen der Mikroelektronik erfüllen. Die Bis-o-amino(thio)-phenole sollen insbesondere die Herstellung gut löslicher Polymer-Vorstufen ermöglichen, die nach der Cyclisierung auf einem Substrat Polybenzoxazole bzw. Polybenzothiazole mit geringer Feuchteaufnahme, hoher Temperaturstabilität und hohem Planarisierungsgrad ergeben. Außerdem sollen die Bis-o-amino(thio)phenole lagerstabil sein und sich bei einer Lagerung an der Luft nicht verändern.

Dies wird erfindungsgemäß durch Bis-o-aminophenole und Bis-o-aminothiophenole folgender Struktur erreicht: 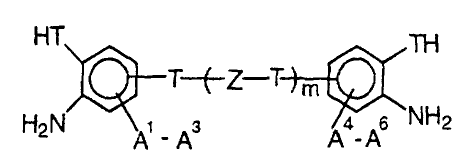 dabei gilt folgendes:
A¹ bis A⁶ sind - unabhängig voneinander - H, F, CH₃, CF₃, OCH₃, OCF₃, CH₂CH₃, CF₂CF₃, OCH₂CH₃ oder OCF₂CF₃,
wobei mindestens einer der Reste A¹ bis A⁶ F oder eine F-haltige Gruppe sein muß;
T ist O oder S, und m ist 0 oder 1;
Z ist einer der folgenden Reste: 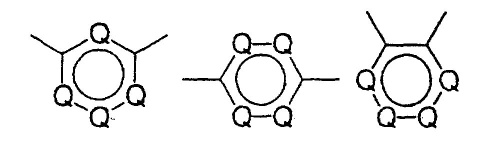 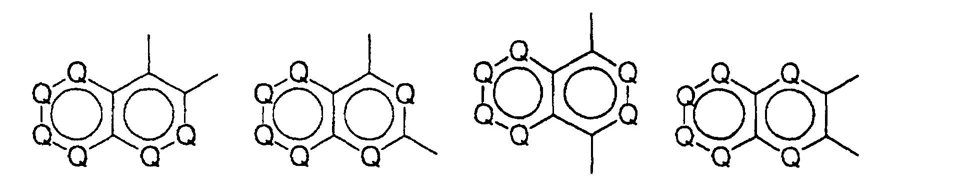 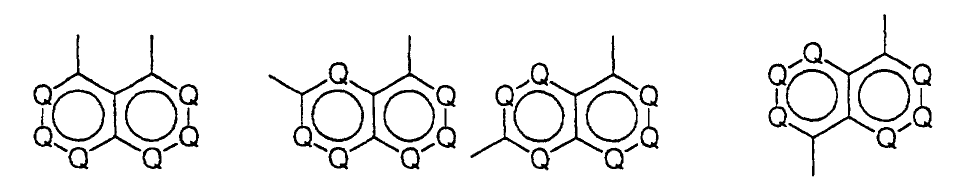 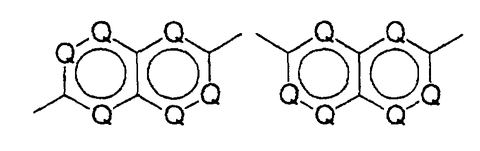 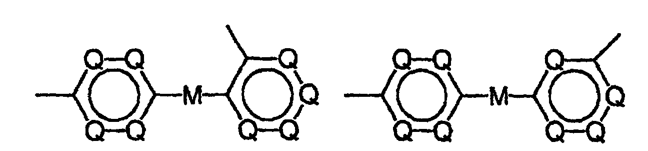 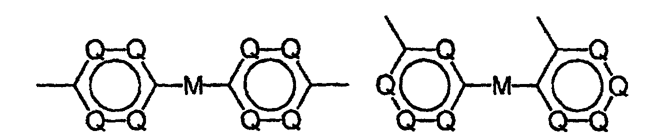 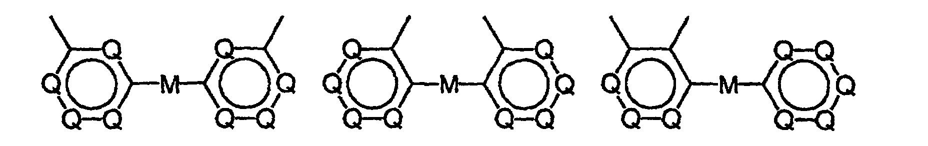 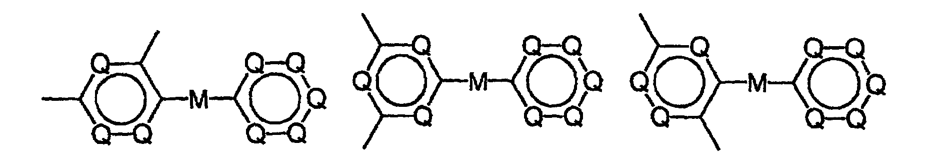 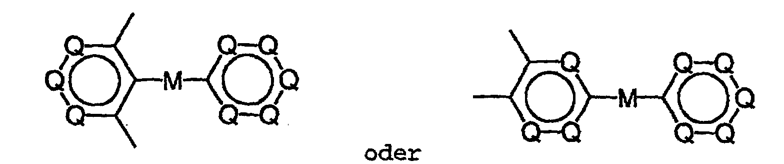 mit
   - Q =: C-A oder N,
   mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl,
   wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
   - M =: eine Einfachbindung, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃)(C₆F₅), C(C₆H₅)₂, C(C₆F₅)₂, CO, SO₂,

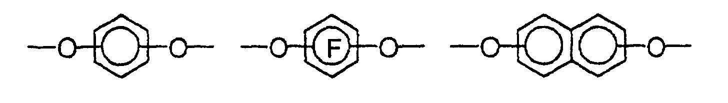

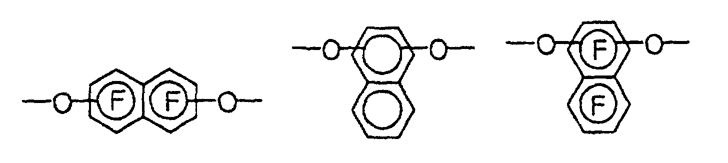

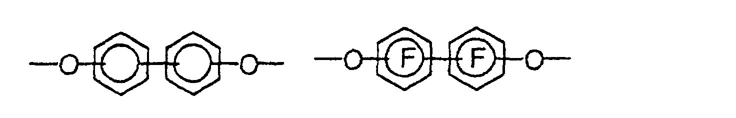

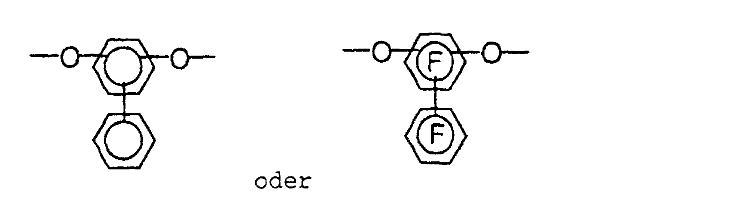

Die neuen Verbindungen weisen beispielsweise folgende Struktur auf:

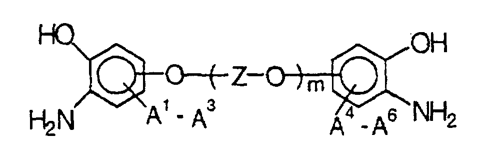

Bei derartigen Verbindungen sind offensichtlich die Etherbrücken für die gute Löslichkeit und die guten Planarisierungseigenschaften der damit hergestellen Polymer-Vorstufen verantwortlich. Im übrigen bedeutet die Charakterisierung "A¹-A³" und "A⁴-A⁶" in der Strukturformel, daß die Aminophenylgruppen Reste A¹, A² und A³ bzw. A⁴, A⁵ und A⁶ aufweisen.

Die Bis-o-amino(thio)phenole werden in der Weise hergestellt, daß
(a) eine Nitroverbindung der Struktur 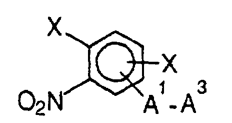 und eine Nitroverbindung der Struktur 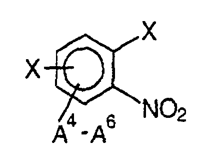 in einem Lösemittel bei einer Temperatur zwischen -10 und 80°C
   entweder mit einem Alkalihydroxid bzw. Alkalihydrogensulfid
   oder - in Gegenwart einer Base - mit einer Dihydroxy-bzw. Dimercaptoverbindung der Struktur

   HT-Z-TH

   oder mit einem Alkalisalz der Dihydroxy- bzw. Dimercaptoverbindung
   zur Reaktion gebracht wird,
   wobei X ein Halogenatom ist und A¹ bis A⁶, T und Z die angegebene Bedeutung haben; und
(b) das dabei gebildete Bis-o-nitro(thio)phenol zum Bis-o-amino(thio)phenol reduziert wird.

Bevorzugt werden Bis-o-amino(thio)phenole hergestellt, bei denen sich die Substituenten A¹ und A⁴, A² und A⁵ sowie A³ und A⁶ jeweils entsprechen und in derselben Position zur Aminogruppe am jeweiligen Phenylrest angeordnet sind. Dies bedeutet, daß bei der Herstellung dieser Verbindungen lediglich eine einzige Nitroverbindung eingesetzt wird.

Die Verbindung HT-Z-TH ist eine aromatische oder substituierte aromatische Verbindung (mit T = O oder S). Für die Umsetzung mit der Nitroverbindung sind prinzipiell alle Verbindungen geeignet, bei denen die Hydroxy- bzw. Mercaptogruppen eine ausreichende Nucleophilie besitzen. Beispiele für derartige Verbindungen sind: Resorcin, Tetrafluorresorcin, Hydrochinon, Tetrafluorhydrochinon, 4,6-Dihydroxypyrimidin, 2,4-Dihydroxy-5-fluorpyrimidin, Octafluorbiphenol, 3,3'-Dihydroxy-2,2'-bipyridyl, 2,2-Bis(4-hydroxyphenyl)-perfluorpropan (6F-Bisphenol A), Bis(4-hydroxyphenyl)-sulfon und 2,6-Dihydroxyanthrachinon.

Die Reaktion zwischen der Dihydroxy- bzw. Dimercaptoverbindung und der Nitroverbindung, bei der Ether- bzw. Thioetherbrücken gebildet werden, erfolgt in Gegenwart einer Base. Diese Base ist vorzugsweise ein Carbonat oder Hydrogencarbonat eines Alkali- oder Erdalkalimetalls, wie Natriumcarbonat und Kaliumcarbonat. Für die (Thio-)etherbildung und den Ersatz des Halogenatoms (in o-Stellung zur Nitrogruppe) durch eine Hydroxy- bzw. Mercaptogruppe sind jeweils mindestens stöchiometrische Mengen der Base erforderlich. Vorteilhaft kann auch eine organische Base mit einem tertiären N-Atom, beispielsweise Triethylamin und Pyridin, eingesetzt werden. In diesem Fall ist die Zugabe von Wasser erforderlich. Anstelle der Dihydroxy- bzw. Dimercaptoverbindung kann auch ein entsprechendes Alkalisalz eingesetzt werden, beispielsweise das Kaliumsalz.

Als Reaktionstemperatur hat sich der Bereich von -10 bis 80°C als geeignet erwiesen. Temperaturen ≤ 80°C werden wegen der höheren Selektivität der Umsetzung bevorzugt. Hierbei liegen die Ausbeuten nämlich nahezu im quantitativen Bereich, was einen deutlichen Vorteil im Vergleich zum Stand der Technik darstellt.

Vorteilhaft wird in der Weise vorgegangen, daß zunächst für einige Zeit, beispielsweise ca. 16 h, eine Temperatur ≤ 25°C eingehalten wird, wobei die Umsetzung der Nitroverbindung mit der Dihydroxy- bzw. Dimercaptoverbindung erfolgt. Anschließend wird die Reaktion bei erhöhter Temperatur, d.h. ≥ 40°C, weitergeführt; hierbei erfolgt dann der Ersatz des Halogenatoms durch eine Hydroxy- bzw. Mercaptogruppe. Bei dieser Vorgehensweise entstehen selektiv Produkte, bei denen sich die Hydroxy- bzw. Mercaptogruppe in o-Stellung zur Nitrogruppe befindet.

Geeignete Lösemittel sind insbesondere Dimethylformamid, Diethylformamid, Dimethylacetamid, Dimethylsulfoxid, N-Methylpyrrolidon, γ-Butyrolacton, Acetonitril, Tetrahydrofuran und Pyridin. Prinzipiell können aber alle polaren aprotischen Lösemittel verwendet werden, in denen die Ausgangsverbindungen löslich sind.

Die Reduktion der Dinitroverbindung führt zum erwünschten Bis-o-amino(thio)phenol. Die Reduktion kann beispielsweise durch Hydrierung mit Wasserstoff an Pd/C durchgeführt werden. Es sind aber prinzipiell alle Verfahren geeignet, die sich für die Reduktion der Nitrogruppe zur Aminogruppe eignen. Die Reduktion erfolgt vorzugsweise bei Temperaturen von 25 bis 50°C. Geeignete Lösemittel sind Ester oder Ether, beispielsweise Essigsäureethylester und Tetrahydrofuran.

Beim Verfahren nach der Erfindung ergeben sich keine der Probleme, die beim Stand der Technik auftreten. Die nach diesem Verfahren hergestellten Bis-o-amino(thio)phenole sind außerdem lagerstabil und können problemlos an der Luft gelagert werden.

Die aus den Bis-o-amino(thio)phenolen nach der Erfindung hergestellten Polymer-Vorstufen sind in vielen organischen Lösemitteln, wie Aceton, Ethyllactat, N-Methylpyrrolidon, Diethylenglykolmono- bzw. -diethylether, Cyclohexanon und γ-Butyrolacton, sowie in metallionenfreien wäßrig-alkalischen Entwicklern gut löslich. Sie sind deshalb als Basispolymer für positiv photostrukturierbare und wäßrig-alkalisch entwickelbare Dielektrika gut geeignet. Die Vorstufen können mittels Schleudertechnik problemlos auf Substrate, wie Siliziumwafer, aufgebracht werden, sie bilden gleichmäßige Filme und lassen sich auf dem Substrat gut cyclisieren. Ein besonderer Vorteil der aus diesen Bis-o-amino(thio)phenolen hergestellten Vorstufen ist das hohe Planarisierungsvermögen und die geringe Feuchteaufnahme.

Anhand von Ausführungsbeispielen soll die Erfindung noch näher erläutert werden.

### Beispiel 1

### Herstellung von 2,2-Bis[4-(4-nitro-3-hydroxy-2,5,6-trifluorphenoxy)-phenyl]-hexafluorpropan

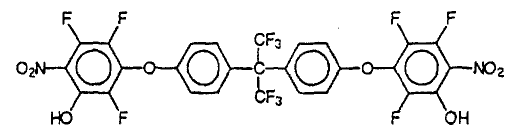

In einem 1l-Dreihalskolben mit Stickstoffzuleitung und Rührer werden 33,6 g 6F-Bisphenol A (0,1 mol) und 42,6 g Pentafluor-nitrobenzol (0,2 mol) in 400 ml Dimethylsulfoxid gelöst. Zur Lösung werden portionsweise 60 g Kaliumcarbonat (0,43 mol) gegeben, dann wird 24 h bei Raumtemperatur gerührt. Anschließend wird in einem regelbaren Ölbad 6 h auf 80°C erhitzt und nach der Zugabe von 10 g Kaliumhydrogencarbonat (0,1 mol) weitere 18 h. Danach läßt man die Reaktionslösung auf Raumtemperatur abkühlen und filtriert den Rückstand über einen Büchnertrichter ab. Nach der Zugabe von 2 l Wasser wird konzentrierte Salzsäure bis zur sauren Reaktion der Lösung zugetropft. Dabei fällt ein gelbes Reaktionsprodukt aus, das über einen Büchnertrichter abfiltriert und dreimal mit Wasser gewaschen wird. Das Reaktionsprodukt wird dann in Ethanol umkristallisiert und anschließend in einem Vakuumschrank 48 h unter Stickstoff bei 40°C/10 mbar getrocknet (Ausbeute: 91 %).

### Charakterisierung:

- Massenspektrum: Molekülpeak bei 718
- Elementaranalyse:
   Theoretischer Wert (in %) : C: 45,1 H: 1,4 N: 3,9
   Ermittelter Wert (in %) : C: 45,1 H: 1,3 N: 3,9
- Schmp.: 70°C.

### Beispiel 2

### Herstellung von 2,2-Bis[4-(4-amino-3-hydroxy-2,5,6-trifluorphenoxy)-phenyl] -hexafluorpropan

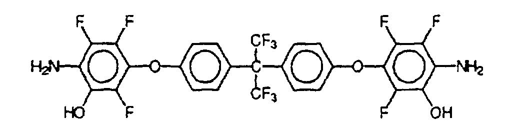

21,5 g des entsprechend Beispiel 1 hergestellten 2,2-Bis[4-(4-nitro-3-hydroxy-2,5,6-trifluor-phenoxy)-phenyl]-hexafluorpropan (0,03 mol) werden in 200 ml eines Gemisches von Tetrahydrofuran und Essigsäureethylester (Volumenverhältnis 1:1) gelöst, und zu der Lösung werden 2 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 2 Tagen wird die Reaktion beendet. Die Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 93 %).

### Charakterisierung:

- Massenspektrum: Molekülpeak bei 658
- Elementaranalyse:
   Theoretischer Wert (in %) : C: 49,3 H: 2,1 N: 4,3
   Ermittelter Wert (in %) : C: 49,1 H: 2,2 N: 4,3

### Beispiel 3

### Herstellung von 1,4-Bis(4-nitro-3-hydroxy-2,5,6-trifluorphenoxy)-tetrafluorbenzol

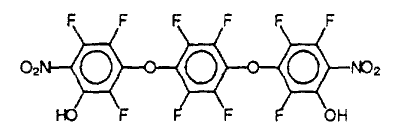

In einem 21-Dreihalskolben mit Stickstoffzuleitung und Rührer werden 18,6 g Tetrafluorhydrochinon (0,1 mol) und 42,6 g Pentafluor-nitrobenzol (0,2 mol) in 400 ml Dimethylsulfoxid gelöst. Zur Lösung werden portionsweise 60 g Kaliumcarbonat (0,43 mol) gegeben, dann wird 24 h bei Raumtemperatur gerührt. Anschließend wird in einem regelbaren Ölbad 4 h auf 60°C erhitzt und nach der Zugabe von 30 g Kaliumhydrogencarbonat (0,3 mol) weitere 6 h. Danach läßt man die Reaktionslösung auf Raumtemperatur abkühlen und filtriert den Rückstand über einen Büchnertrichter ab. Nach der Zugabe von 500 ml Wasser und 300 ml Essigsäureethylester wird konzentrierte Salzsäure bis zur sauren Reaktion der Lösung zugetropft. Die organische Phase wird dann dreimal mit Wasser gewaschen, über Natriumsulfat getrocknet und in einem Rotationsverdampfer auf die Hälfte eingeengt. Nach 2 Tagen werden die ausgefallenen gelben Kristalle abfiltriert, mit Methylenchlorid gewaschen und in einem Vakuumschrank 48 h unter Stickstoff bei 40°C/10 mbar getrocknet (Ausbeute: 93 %).

### Charakterisierung:

- Massenspektrum: Molekülpeak bei 564
- Elementaranalyse:
   Theoretischer Wert (in %) : C: 38,3 H: 0,4 N: 5,0
   Ermittelter Wert (in %) : C: 38,4 H: 0,3 N: 4,9
- Schmp.: 234°C (Zersetzung).

### Beispiel 4

### Herstellung von 1,4-Bis(4-amino-3-hydroxy-2,5,6-trifluorphenoxy)-tetrafluorbenzol

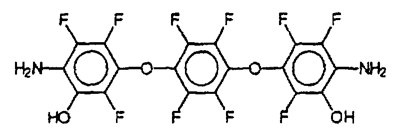

50 g des entsprechend Beispiel 3 hergestellten 1,4-Bis-(4-nitro-3-hydroxy-2,5,6-trifluor-phenoxy)-tetrafluorbenzol (0,09 mol) werden in 500 ml eines Gemisches von Tetrahydrofuran und Essigsäureethylester (Volumenverhältnis 1:1) gelöst, und zu der Lösung werden 5 g Pd/C (Palladium/Kohlenstoff) gegeben. Dann wird bei Raumtemperatur in einem Autoklaven unter starkem Rühren mit Wasserstoff bei einem Druck von 1bar hydriert; nach 2 Tagen wird die Reaktion beendet. Die gelbe Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 92 %).

### Charakterisierung:

- Massenspektrum: Molekülpeak bei 504
- Elementaranalyse:
   Theoretischer Wert (in %) : C: 42,9 H: 1,2 N: 5,6
   Ermittelter Wert (in %) : C: 41,7 H: 1,3 N: 5,7

### Beispiel 5

### Herstellung von 4,6-Bis(4-nitro-3-hydroxy-2,5,6-trifluorphenoxy)-pyrimidin

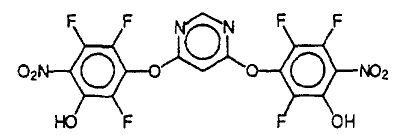

In einem 21-Dreihalskolben mit Stickstoffzuleitung und Rührer werden 11,2 g 4,6-Dihydroxypyrimidin (0,1 mol) und 42,6 g Pentafluor-nitrobenzol (0,2 mol) in 400 ml Dimethylsulfoxid gelöst. Zur Lösung werden portionsweise 60 g Kaliumcarbonat (0,43 mol) gegeben, dann wird 24 h bei Raumtemperatur gerührt. Anschließend wird in einem regelbaren Ölbad 4 h auf 60°C erhitzt und nach der Zugabe von 30 g Kaliumhydrogencarbonat (0,3 mol) weitere 6 h. Danach läßt man die Reaktionslösung auf Raumtemperatur abkühlen und filtriert den Rückstand über einen Büchnertrichter ab. Nach der Zugabe von 500 ml Wasser und 300 ml Essigsäureethylester wird konzentrierte Salzsäure bis zur sauren Reaktion der Lösung zugetropft. Die organische Phase wird dann dreimal mit Wasser gewaschen, über Natriumsulfat getrocknet und in einem Rotationsverdampfer auf die Hälfte eingeengt. Nach 2 Tagen werden die ausgefallenen orangebraunen Kristalle abfiltriert, mit Petroleumbenzin gewaschen und in einem Vakuumschrank 48 h unter Stickstoff bei 40°C/10 mbar getrocknet (Ausbeute: 94 %).

### Charakterisierung:

- Massenspektrum: Molekülpeak bei 494
- Elementaranalyse:
   Theoretischer Wert (in %) : C: 38,9 H: 0,8 N: 11,3
   Ermittelter Wert (in %) : C: 39,1 H: 0,7 N: 11,1

### Beispiel 6

### Herstellung von 4,6-Bis(4-amino-3-hydroxy-2,5,6-trifluorphenoxy)-pyrimidin

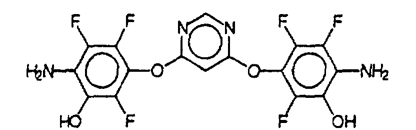

50,8 g des entsprechend Beispiel 5 hergestellten 4,6-Bis-(4-nitro-3-hydroxy-2,5,6-trifluor-phenoxy)-pyrimidin (0,12 mol) werden in 500 ml eines Gemisches von Tetrahydrofuran und Essigsäureethylester (Volumenverhältnis 1:1) gelöst, und zu der Lösung werden 5 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 2 Tagen wird die Reaktion beendet. Die gelbe Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 93 %).

### Charakterisierung:

- Massenspektrum: Molekülpeak bei 434
- Elementaranalyse:
   Theoretischer Wert (in %) : C: 44,3 H: 1,9 N: 12,9
   Ermittelter Wert (in %) : C: 44,3 H: 1,8 N: 12,8

### Beispiel 7

### Herstellung von 4,4'-Bis(4-nitro-3-hydroxy-2,5,6-trifluorphenoxy)-octafluorbiphenyl

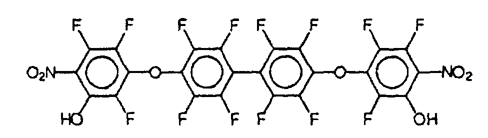

In einem 21-Dreihalskolben mit Stickstoffzuleitung und Rührer werden 33 g 4,4'-Octafluorbiphenol (0,1 mol) und 42,6 g Pentafluor-nitrobenzol (0,2 mol) in 400 ml Dimethylsulfoxid gelöst. Zur Lösung werden portionsweise 60 g Kaliumcarbonat (0,43 mol) gegeben, dann wird 24 h bei Raumtemperatur gerührt. Anschließend wird in einem regelbaren Ölbad 48 h auf 50°C erhitzt, dann läßt man die Reaktionslösung auf Raumtemperatur abkühlen und filtriert den Rückstand über ein Faltenfilter ab. Nach der Zugabe von 500 ml Wasser und 300 ml Essigsäureethylester wird konzentrierte Salzsäure bis zur sauren Reaktion der Lösung zugetropft. Die organische Phase wird dann dreimal mit Wasser gewaschen, über Natriumsulfat getrocknet und in einem Rotationsverdampfer auf die Hälfte eingeengt. Nach 2 Tagen werden die ausgefallenen gelben Kristalle abfiltriert, mit einem Gemisch von Methylenchlorid und Petroleumbenzin (Volumenverhältnis 1:1) gewaschen und in einem Vakuumschrank 48 h unter Stickstoff bei 40°C/10 mbar getrocknet (Ausbeute: 90 %).

### Charakterisierung:

- Massenspektrum: Molekülpeak bei 712
- Elementaranalyse:
   Theoretischer Wert (in %) : C: 40,5 H: 0,3 N: 3,9
   Ermittelter Wert (in %) : C: 40,7 H: 0,4 N: 3,8
- Schmp.: > 300°C.

### Beispiel 8

### Herstellung von 4,4'-Bis(4-amino-3-hydroxy-2,5,6-trifluorphenoxy)-octafluorbiphenyl

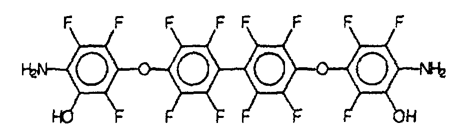

49,8 g des entsprechend Beispiel 7 hergestellten 4,4'-Bis-(4-nitro-3-hydroxy-2,5,6-trifluor-phenoxy)-octafluorbiphenyl (0,07 mol) werden in 500 ml eines Gemisches von Tetrahydrofuran und Essigsäureethylester (Volumenverhältnis 1:1) gelöst, und zu der Lösung werden 5 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 2 Tagen wird die Reaktion beendet. Die gelbe Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 90 %).

### Charakterisierung:

- Massenspektrum: Molekülpeak bei 652
- Elementaranalyse:
   Theoretischer Wert (in %) : C: 44,2 H: 0,9 N: 4,3
   Ermittelter Wert (in %) : C: 44,0 H: 0,8 N: 4,4

## Patentansprüche

1. Bis-o-aminophenole und Bis-o-aminothiophenole der Struktur 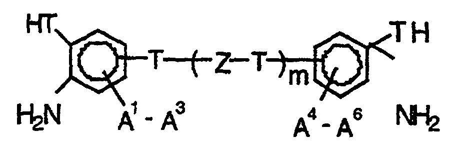 wobei folgendes gilt:
A¹ bis A⁶ sind - unabhängig voneinander - H, F, CH₃, CF₃, OCH₃, OCF₃, CH₂CH₃, CF₂CF₃, OCH₂CH₃ oder OCF₂CF₃,
wobei mindestens einer der Reste A¹ bis A⁶ F oder eine F-haltige Gruppe sein muß;
T ist O oder S, und m ist 0 oder 1;
Z ist einer der folgenden Reste: 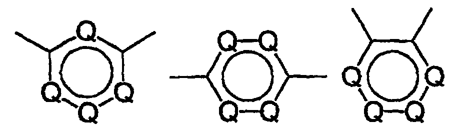 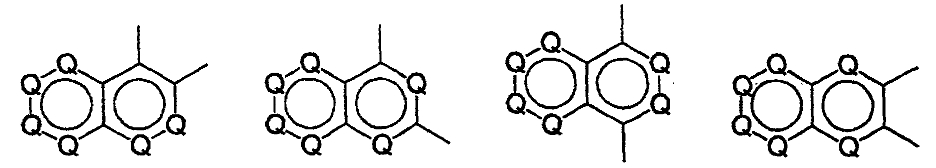 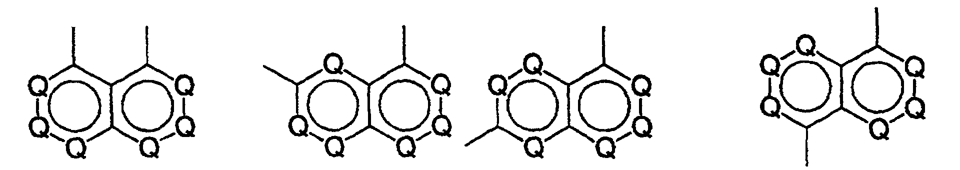 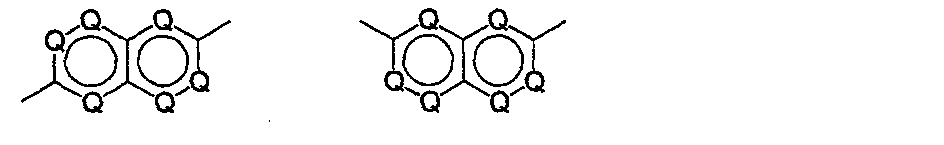 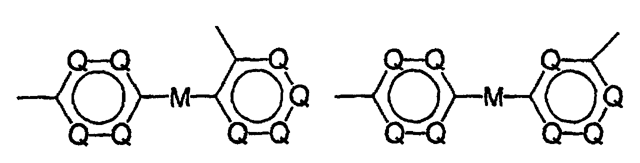 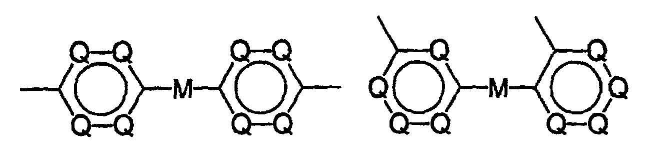 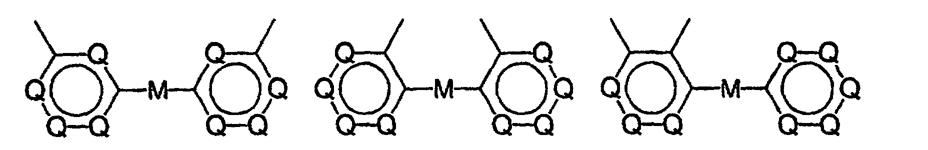 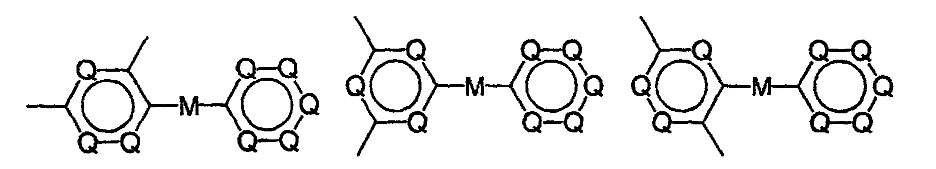 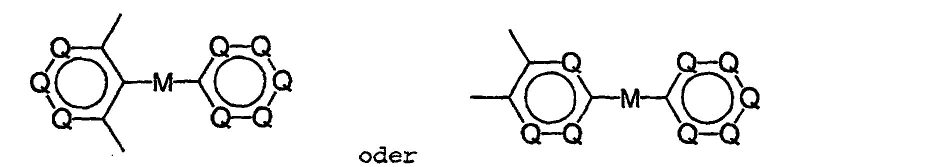 mit
Q = C-A oder N,
mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = 0 bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl,
wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
M = eine Einfachbindung, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃) (C₆H₅), C(CH₃) (C₆F₅), C(CF₃) (C₆H₅), C(CF₃) (C₆F₅), C(C₆H₅)₂, C(C₆F₅)₂, CO, SO₂,

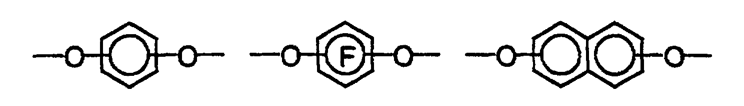

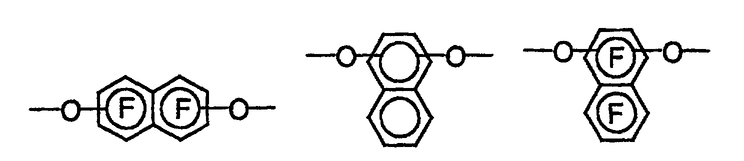

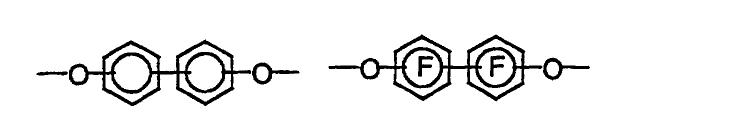

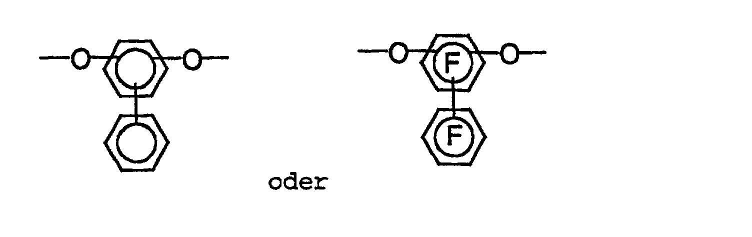

2. Verfahren zur Herstellung von Bis-o-aminophenolen und Bis-o-aminothiophenolen nach Anspruch 1, **dadurch gekennzeichnet, daß**
(a) eine Nitroverbindung der Struktur 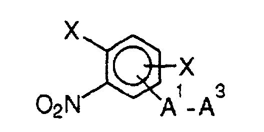 und eine Nitroverbindung der Struktur 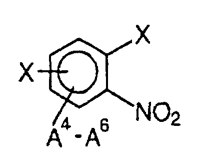 in einem Lösemittel bei einer Temperatur zwischen -10 und 80°C
entweder mit einem Alkalihydroxid bzw. Alkalihydrogensulfid
oder - in Gegenwart einer Base - mit einer Dihydroxy-bzw. Dimercaptoverbindung der Struktur
HT-Z-TH
oder mit einem Alkalisalz der Dihydroxy- bzw. Dimercaptoverbindung
zur Reaktion gebracht wird,
wobei X ein Halogenatom ist und A¹ bis A⁶, T und Z die angegebene Bedeutung haben; und
(b) das dabei gebildete Bis-o-nitro(thio)phenol zum Bis-o-amino(thio)phenol reduziert wird.

3. Verfahren nach Anspruch 2, **dadurch gekennzeichnet, daß** als Base ein Carbonat oder Hydrogencarbonat eines Alkali- oder Erdalkalimetalls eingesetzt wird.

4. Verfahren nach Anspruch 2, **dadurch gekennzeichnet, daß** eine organische Base mit einem tertiären N-Atom zusammen mit Wasser eingesetzt wird.

5. Verfahren nach einem der Ansprüche 2 bis 4, d a**durch gekennzeichnet**, daß die Reduktion durch Wasserstoff erfolgt und mit Pd/C katalysiert wird.

## Claims

1. Bis-o-aminophenols and bis-o-aminothiophenols of the structure 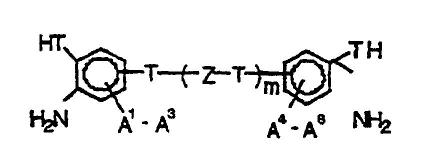 where
A¹ to A⁶ are - independently of one another - H, F, CH₃, CF₃, OCH₃, OCF₃, CH₂CH₃, CF₂CF₃, OCH₂CH₃ or OCF₃CF₃,
where at least one of the radicals A¹ to A⁶ must be F or an F-containing group;
T is O or S, and m is 0 or 1;
Z is one of the following radicals: 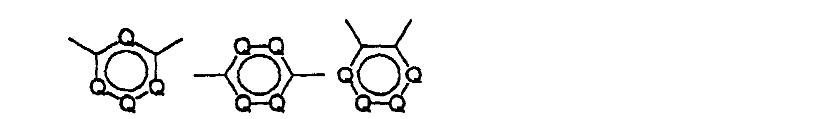 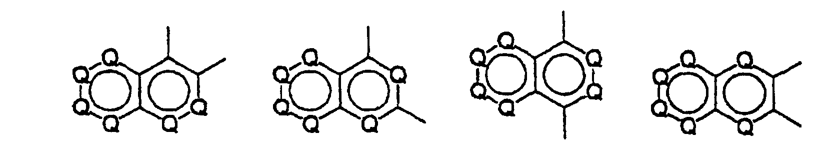 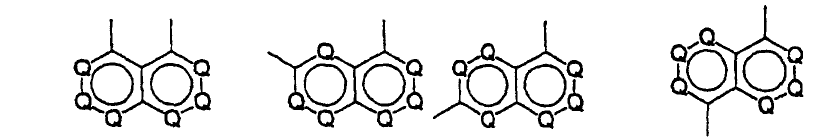 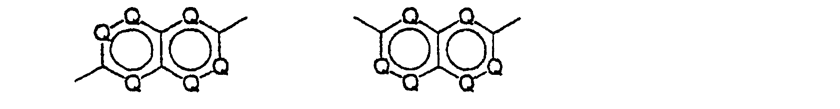 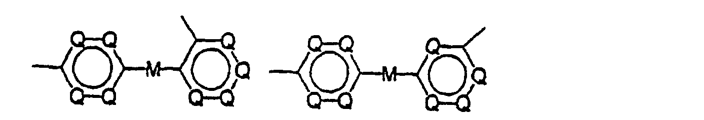 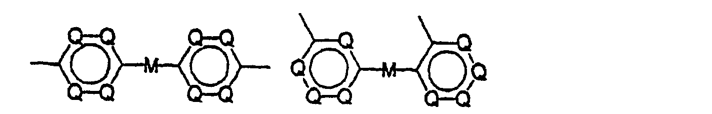 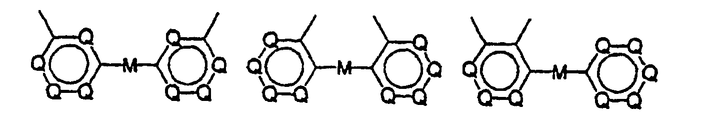 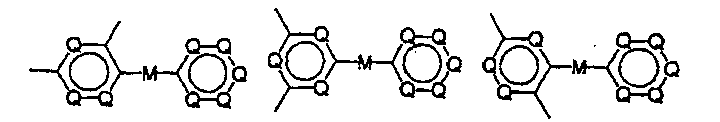 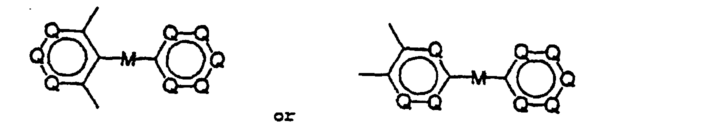 where
Q = C-A or N, where A = H, F, (CH₂)ₚCH₃, (CF₂)ₚCF₃, O(CH₂)ₚCH₃, O(CF₂)ₚCF₃, CO (CH₂)ₚCH₃, CO (CF₂)ₚCF₃ where p = 0 to 8 (chain linear or branched), OC(CH₃)₃, OC(CF₃)₃, C₆H₅, C₆F₅, OC₆H₅, OC₆F₅, cyclopentyl, perfluorocyclopentyl, cyclohexyl or perfluorocyclohexyl,
where, in the isolated aromatic rings, at most 3 N atoms may be present per ring and only 2 N atoms can be adjacent, and in the fused ring systems, at most 2 N atoms may be present per ring,
M = a single bond, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C (CH₃) (C₆H₅), C (CH₃) (C₆F₅), C(CF₃) (C₆H₅), C (CF₃) (C₆F₅), C (C₆H₅)₂, C(C₆F₅)₂, CO, SO₂,

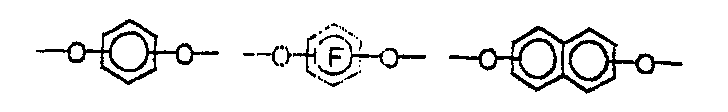

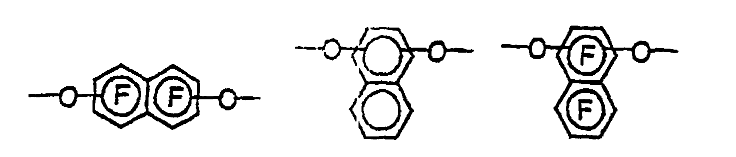

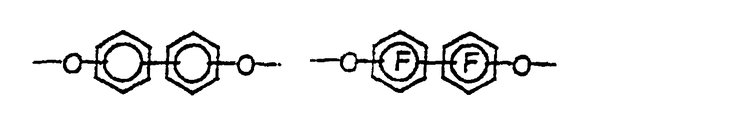

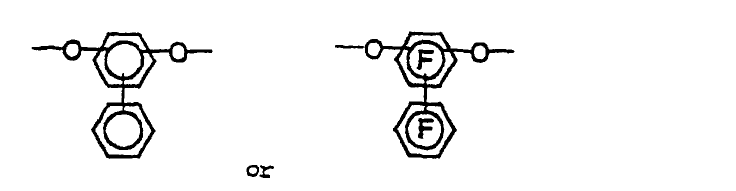

2. Process for the preparation of bis-o-aminophenols and bis-o-aminothiophenols according to Claim 1,
**characterized in that**
(a) a nitro compound of the structure 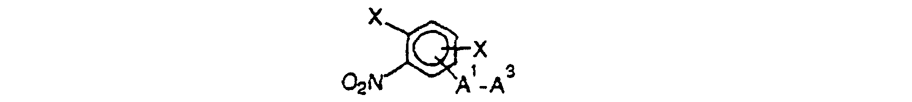 and a nitro compound of the structure 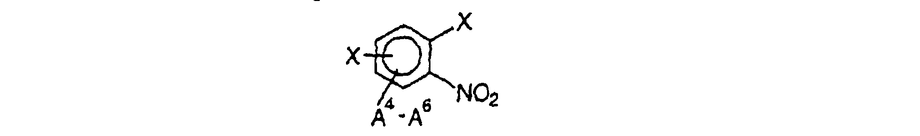 in a solvent at a temperature between -10 and 80°C is reacted either with an alkali metal hydroxide or alkali metal hydrogensulphide or - in the presence of a base - with a dihydroxy or dimercapto compound of the structure
HT-Z-TH
or with an alkali metal salt of the dihydroxy or dimercapto compound,
where X is a halogen atom, and A¹ to A⁶, T and Z have the meanings given; and
(b) the bis-o-nitro(thio)phenol formed therein is reduced to the bis-o-amino(thio)phenol.

3. Process according to Claim 2, **characterized in that** the base used is a carbonate or hydrogencarbonate of an alkali metal or alkaline earth metal.

4. Process according to Claim 2, **characterized in that** an organic base with a tertiary N atom is used together with water.

5. Process according to any one of Claims 2 to 4, **characterized in that** the reduction takes place by means of hydrogen and is catalysed with Pd/C.

## Revendications

1. Bis-o-aminophénols et bis-o-aminothiophénols de formule : 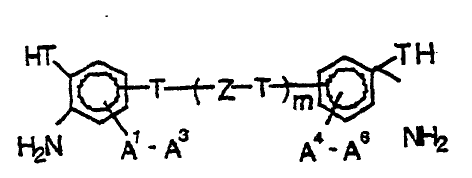 dans laquelle :
A¹ à A⁶ sont indépendamment les uns des autres H, F, CH₃, CF₃, OCH₃, OCF₃, CH₂CH₃, CF₂CF₃, OCH₂CH₃ ou OCF₂CF₃,
au moins l'un des radicaux A¹ à A⁶ doit être F ou un groupe contenant F,
T est O ou S et m est 0 ou 1 ;
Z est l'un des radicaux suivants : 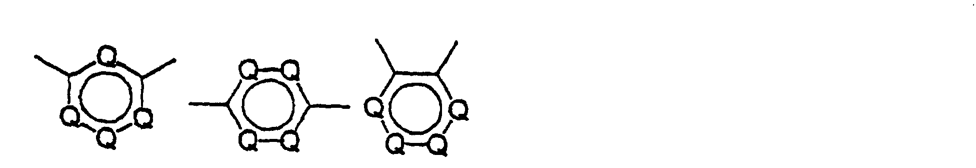 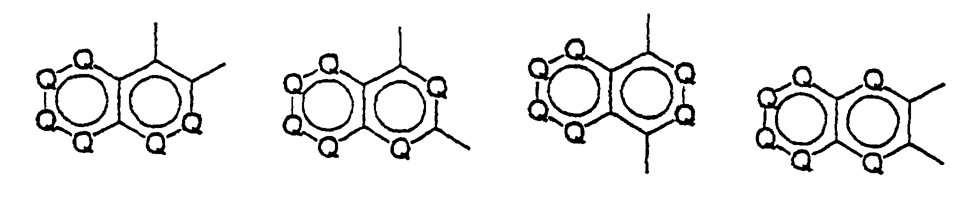 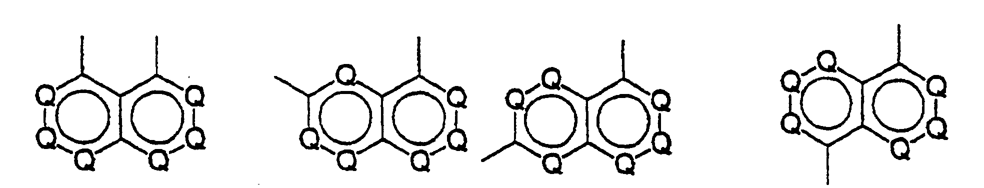 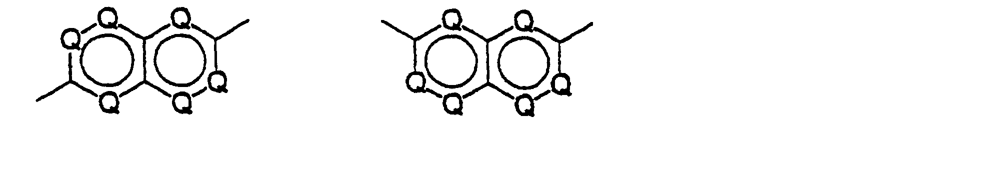 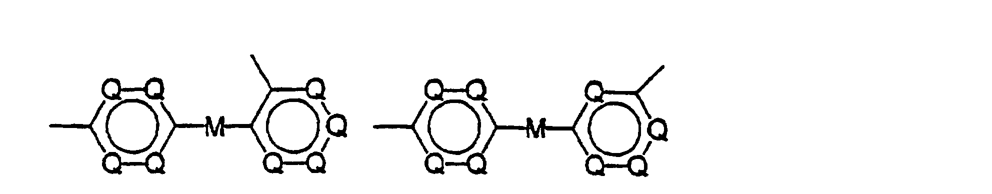 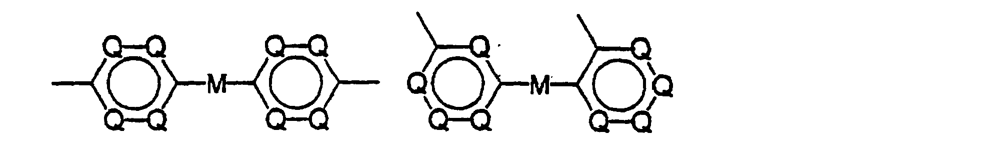 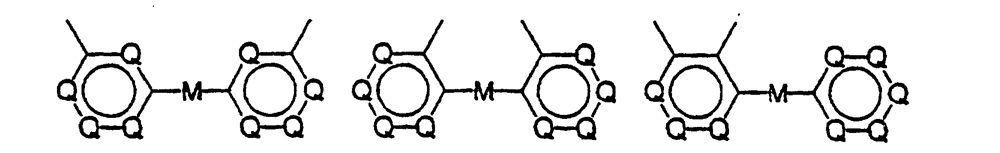 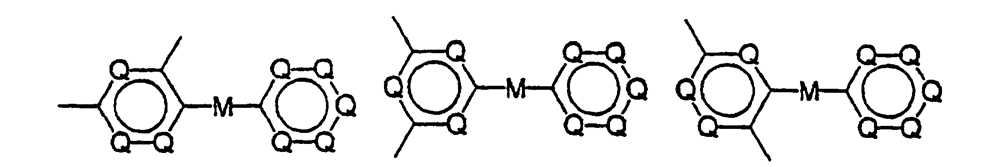 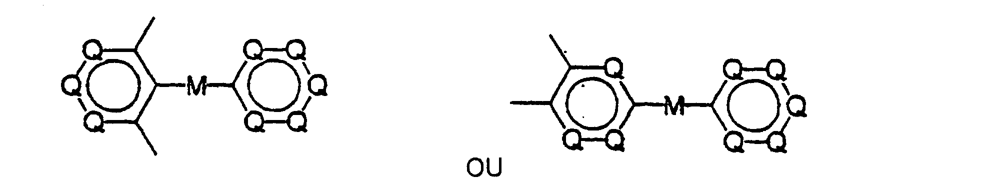
où Q = C-A ou N,
avec A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ avec p = 0 à 8 (chaîne linéaire ou ramifiée), OC(CH₃)₃, OC(CF₃)₃, C₆H₅, C₆F₅, OC₆H₅, OC₆F₅, cyclopentyle, perfluorocyclopentyle, cyclohexyle ou perfluorocyclohexyle, dans lesquels il peut y avoir dans les cycles aromatiques isolés au maximum 3 atomes d'azote par cycle et seulement 2 atomes d'azote peuvent être voisins et dans les systèmes cycliques condensés il peut y avoir au maximum 2 atomes d'azote par cycle,
M = une simple liaison, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃)(C₆F₅), C(C₆H₅)₂, C(C₆F₅)₂, CO, SO₂,

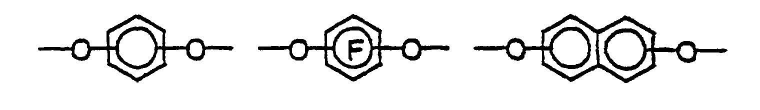

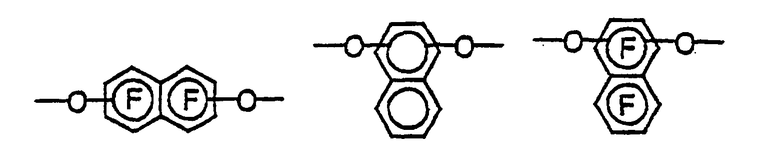

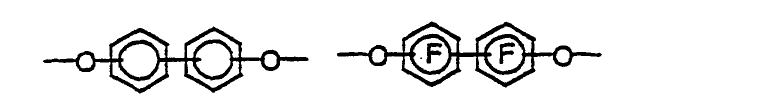

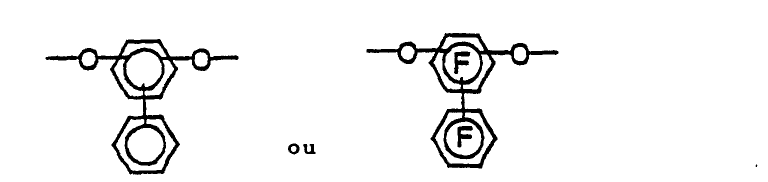

2. Procédé de préparation de bis-o-aminophénol et de bis-o-thiophénol suivant la revendication 1, **caractérisé en ce qu'**il consiste à mettre
(a) un composé nitro de formule 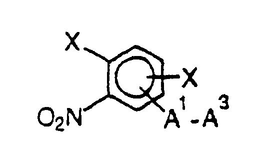 et un composé nitro de formule 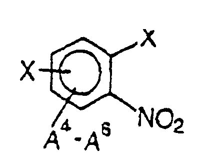 à réagir dans un solvant à une température comprise entre -10 et 80°C soit sur un hydroxyde de métal alcalin ou sur un hydrogénosulfure de métal alcalin,
soit en présence d'une base, sur un composé dihydroxy ou dimercapto de formule
HT-Z-TH
soit sur un sel de métal alcalin du composé dihydroxy ou du composé dimercapto
X étant un atome d'halogène et A¹ à A⁶, T et Z ayant la signification indiquée ; et
(b) à réduire le bis-o-nitro(thio)phénol ainsi formé en le bis-o-amino(thio)phénol.

3. Procédé suivant la revendication 2, **caractérisé en ce qu'**il consiste à utiliser comme base un carbonate ou un hydrogénocarbonate d'un métal alcalin ou d'un métal alcalinoterreux.

4. Procédé suivant la revendication 2, **caractérisé en ce qu'**il consiste à utiliser une base organique ayant un atome d'azote tertiaire en même temps que de l'eau.

5. Procédé suivant l'une des revendications 2 à 4, **caractérisé en ce qu'**il consiste à effectuer la réaction par de l'hydrogène et à catalyser par Pd/C.
